# EUROPEAN PATENT SPECIFICATION

(11) **EP 2 236 487 B1**
(45) Date of publication and mention of the grant of the patent: **02.09.2015**
(21) Application number: 10156421.9
(22) Date of filing: 12.03.2010
(51) Int. Cl.: C07C 45/74, C07C 47/225

(54) **Processes for producing levosandal**
Verfahren zur Herstellung von Levosandal
Procédé de production de levosandal

(30) Priority: 02.04.2009 EP 09382045
(43) Date of publication of application: 06.10.2010
(73) Proprietor: INTERNATIONAL FLAVORS & FRAGRANCES INC., New York New York 10019 (US)
(72) Inventor: Corma Canos, Avelino, 46020 Valencia (ES); Iborra Jornet, Sara, 46006 Valencia (ES); Velty, Alexandra, 46018 Valencia (ES)
(74) Representative: Lawrence, John

(56) References cited:
- EP-A1- 0 838 451
- WO-A1-01/38278
- US-A- 4 318 831
- RUIZ ET AL: "Meerwein-Ponndorf-Verley reaction of acetophenones with 2-propanol over MgAl mixed oxide: The substituent effect" CATALYSIS COMMUNICATIONS, ELSEVIER SCIENCE, AMSTERDAM, NL LNKD- DOI:10.1016/J.CATCOM.2006.10.007, vol. 8, no. 7, 1 July 2007 (2007-07-01), pages 1036-1040, XP022071199 ISSN: 1566-7367
- RUIZ J R ET AL: "Reduction of ketones and aldehydes to alcohols with magnesium-aluminium mixed oxide and 2-propanol" JOURNAL OF MOLECULAR CATALYSIS. A, CHEMICAL, ELSEVIER, AMSTERDAM, NL LNKD- DOI:10.1016/J.MOLCATA.2005.11.002, vol. 246, no. 1-2, 1 March 2006 (2006-03-01), pages 190-194, XP025156120 ISSN: 1381-1169 [retrieved on 2006-03-01]

## Description

### FIELD OF THE INVENTION

The present invention relates to processes for producing Levosandal (2-ethyl-4-(2,2,3-trimethyl-3-cyclopenten-1-yl)-2-butenal) and Levosandol (2-ethyl-4-(2,2,3-trimethyl-3-cyclopenten-1-yl)-2-buten-1-ol) using heterogeneous bifunctional catalysts.

### BACKGROUND OF THE INVENTION

α,β-Unsaturated aldehyde compounds as well as α,β-unsaturated alcohols are useful compounds as perfumes, perfume precursors or intermediates for pharmaceuticals and agricultural chemicals.

One of the compounds which refer the present invention 2-ethyl-4-(2,2,3-trimethyl-3-cyclopenten-1-yl)-2-butenal is produced by the cross aldol condensation between campholenal and butanal in the presence of know homogeneous and heterogeneous aldol condensation catalysts.

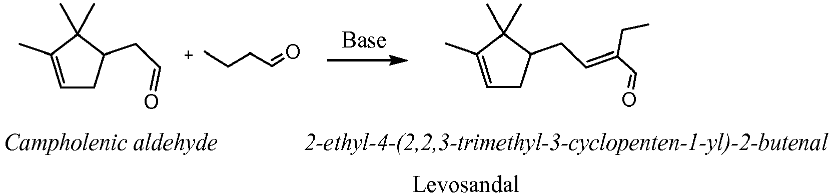

In the patent US 4318831 (1982) Klein et al. describe a method for producing cyclopentene derivatives with application to the perfume preparation. Among the examples it is described the preparation of compounds by cross-aldol condensation between campholenal and aldehydes such as propanaldehyde and butyraldehyde using sodium ethoxyde as catalyst (10 wt% with respect to campholenal) at 0 °C. The molar ratio aldehyde/campholenal is 2 and the aldehyde is dropped to campholenal to gradually react with each other. The neutralization of the catalysts is carried out with acetic acid and after distillation of the reaction mixture the α,β-unsaturated aldehyde is obtained in 60-80 % yield. In a more recent patent application, Ishida el al. (WO 2007/063703 , 2007) describes a process for preparing α,β-unsaturated aldehyde compounds, among them 2-ethyl-4-(2,2,3-trimethyl-3-cyclopenten-1-yl)-2-butenal, by the cross-aldol condensation between camphonelal and butanal in the presence of an amine and a protonic acid having 4 to 20 carbons atoms or a salt thereof. The catalyst is neutralized at the end of the reaction using acetic acid. In general, the use of homogeneous catalysts involves the necessity of a neutralization step and the requirement of a thorough wash with water of the final reaction mixtures. Moreover, in some cases, self condensation of the aldehydes involved also occurs, giving by-products which have to be eliminated by distillation. Besides, the distillation process leads to isomerization and polymerization reactions of the desired compounds decreasing the final yield. In order to overcome these inconveniencies, the use of heterogeneous catalysts appears as an interesting alternative for the production of α,β-unsaturated aldehydes. Patent Application WO 01/38278 (2001) describes a process for preparing α,β-unsaturated carbonyl compounds (among them 2-ethyl-4-(2,2,3-trimethyl-3-cyclopenten-1-yl)-2-butenal) using heterogeneous catalysts having basic characteristics. The catalysts able to perform the condensation between campholenal and butanal are among alkaline earth oxides, layered double hydroxides (LDH) of divalent and trivalent metals in their calcined form (mixed oxides) and hydrated form of these mixed oxides.

Levosandol is produced by reduction of the aldehyde precursor compound by different methods. For instance, in the patent documents WO2002022526 (2002) and EP 829463 (1998) are described the selective reduction of carbonyl compounds through a hydrogenation process using ruthenium phosphine complexes and where Levosandol is prepared by hydrogenation of the aldehyde precursor compound. In another patent the hydrogenation is performed using a Cu chromite catalyst (WO 9310068 (1993). In other patents the reduction is performed using hydride metal salts such as NaBH₄ (DE 3441902, 1986;EP 155591, 1985; WO 9310068, 1993; and WO 2007/063703, 2007). Finally, the selective reduction of the carbonyl group can be carried out through the Meerwein-Ponndorf-Verley (MPV) reaction using aluminium alcoholates as catalysts (WO 9732836, 1997). Thus, in Patent JP 55139330 (1980) the condensation between campholenal and butanal is performed using KOH in MeOH giving the α,β-unsaturated aldehyde which is purified and subsequently reduced with aluminium isopropoxide in the presence of isopropanol to Levosandal in a yield of 70 %.

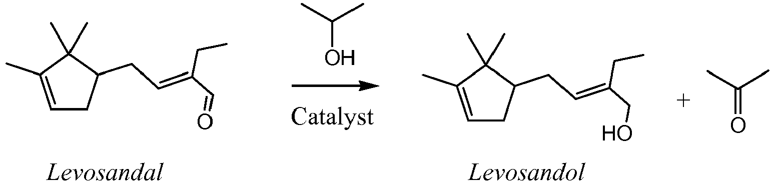

### SUMMARY OF THE INVENTION

In the present work we have surprisingly found that working with hydrated mixed oxides in the condensation between campholenal and butanal, the yield and selectivity to 2-ethyl-4-(2,2,3-trimethyl-3-cyclopenten-1-yl)-2-butenal is notably enhanced if a controlled amount of an aliphatic alcohol is added onto the catalyst previously to the reaction. Therefore, in this invention we present a new method for producing 2-ethyl-4-(2,2,3-trimethyl-3-cyclopenten-1-yl)-2-butenal in high yield and selectivity which involves the condensation of campholenal and butanal in the presence of a hydrated mixed oxide derived from LDH of divalent a trivalent metals in where a controlled amount of an aliphatic alcohol is added at the beginning of the reaction over the catalyst.

In accordance with the present invention, there is also provided a process for producing the unsaturated alcohol derivative of the 2-ethyl-4-(2,2,3-trimethyl-3-cyclopenten-1-yl)-2-butenal, this is Levosandol or Bacdanol (2-ethyl-4-(2,2,3-trimethyl-3-cyclopenten-1-yl)-2-buten-1-ol). Bacdanol is suitable as a synthetic perfume having a sandalwood-like scent.

In the present invention we present a process for producing levosandol starting from Levosandal using a heterogeneous bifunctional catalyst through the Meeerwein-Ponndorf-Verley reduction of Levosandal in the presence of a secondary alcohol as reducing agent. Moreover by coupling the condensation reaction between campholenal and butanal with the MPV reaction, we can obtain Levosandal through a one pot cascade process using the same bifunctional catalysts. The catalysts possess Lewis acid and basic sites, in such a way that it is able to catalyze as the first step, the aldol condensation between campholenal and butanal which yield the 2-ethyl-4-(2,2,3-trimethyl-3-cyclopenten-1-yl)-2-butenal. The aldehyde is reduced to the corresponding alcohol through the MPV reaction by the addition of a secondary alcohol in this case catalyzed by the Lewis acid and basic sites.

### DETAILED DESCRIPTION OF THE INVENTION

The present invention relates to a process for producing 2-ethyl-4-(2,2,3-trimethyl-3-cyclopenten-1-yl)-2-butenal useful as perfume or intermediate for perfume with high yield and selectivity by the cross-aldol condensation between campholenal and butanal in the presence of a controlled amount of a primary or secondary aliphatic alcohol with 1 to 12 carbon atoms using a bifunctional heterogeneous catalyst, wherein the controlled amount of an aliphatic alcohol is between 0.3 and 8 g of aliphatic alcohol per gram of catalyst.

Also described is a process for producing Levosandol (2-ethyl-4-(2,2,3-trimethyl-3-cyclopenten-1-yl)-2-buten-1-ol) useful as perfume, starting from Levosandal through a MPV reduction using a bifunctional heterogeneous catalysts, as well as a process for producing levosandol (2-ethyl-4-(2,2,3-trimethyl-3-cyclopenten-1-yl)-2-buten-1-ol) through one pot cascade process which involves as the first step the aldol condensation between campholenal and butanal followed by the MPV reaction in the presence of a secondary alcohol and a bifunctional heterogeneous catalyst.

The synthesis of 2-ethyl-4-(2,2,3-trimethyl-3-cyclopenten-1-yl)-2-butenal is performed by mixing the reactants with a heterogeneous catalyst which has been treated previously by adding a controlled amount of an aliphatic alcohol. We have found that the addition of this controlled amount of the aliphatic alcohol is essential in order to obtain high conversion and selectivity to the desired compound.

The aliphatic alcohol can be selected between primary and secondary alcohols with 1 to 12 carbon atoms. The amount of aliphatic alcohol added is between 0.3 to 8 g of alcohol per gram of solid catalyst. Higher amounts of the aliphatic alcohol added over the catalyst showed to be detrimental for the reaction performance.

The condensation reaction between campholenal and butanal is performed preferentially using a molar ratio campholenal/butanal between 1:1 and 1:10.

The amount of catalyst can be between 1 and 40 wt% with respect the total amount of reactants.

The use of solvent (except the amount of aliphatic alcohol required) in the above condensation is not necessary but optative. Also, the removal of water produced during the reaction is not particularly required.

The condensation can be carried out by mixing the reactants to react or by dropping the butanal over the mixture campholenal-catalyst slurry to gradually react with each of other.

The aldol condensation can be carried out under inert atmosphere or in the presence of air, in a batch reactor or in a fixed bed reactor.

In optimum reaction conditions, the procedure of the invention allows to obtain a conversion of campholenal higher 98 % with selectivity to 2-ethyl-4-(2,2,3-trimethyl-3-cyclopenten-1-yl)-2-butenal higher than 95 %.

The condensation reaction can be carried out in a continuous reactor or in a batch reactor. The reaction can be performed under inert atmosphere, at atmospheric pressure or in autoclave reactor at pressures between 101325 and 2020500 Pascals (Pa) (i.e., between 1 and 20 atm) and a temperature between 25 and 150°C, preferentially between 30 and 80 °C.

Concerning to the reduction to Levosandal to Bacdanol through the Merweein-Ponndorf-Verley reaction the reaction is performed in the presence of a secondary alcohol as for instance isopropanol or 2-butanol which acts as reducing agent and as solvent. The molar ratio of secondary alcohol/ Levosandal is between 10:1 to 50: 1, preferentially between 20:1 and 30:1.

The ratio (in weight) of heterogeneous catalyst/ Levosandal is between 1.6 to 0.5, preferentially between 1.0 to 0.8.

The reduction reaction can be carried out in continuous reactor or in a batch reactor. The reaction can be performed under inert atmosphere at atmospheric pressure or in autoclave reactor at pressures between 1 and 20 atm and a temperature between 25 and 150 °C, preferentially between 60 and 90 °C.

The bacdanol (2-ethyl-4-(2,2,3-trimethyl-3-cyclopenten-1-yl)-2-buten-1-ol)) can be prepared through a one pot cascade process which involves as the first step the aldol condensation between campholenal and butanal in the presence of the heterogeneous catalysts. When the campholenal achieves preferentially above 90 % of conversion, the reducing alcohol is added if necessary, and the reaction variables are adjusted for the MPV reaction occurs on the bifunctional heterogeneous catalyst giving bacdanol.

The starting solid which refers the present invention which are used both in the first step condensation reaction and the second step MPV reduction are mixed oxides or hydrated mixed oxides derived of LDH of divalent and trivalent metals (M⁺²/N⁺³) of formula:

MₘNₙ(OH)₂ₘ₊₂ₙ(A)ₐbH₂O,

Where M is a divalent metal cation selected between Mg⁺², Ni⁺², Zn⁺², Sn⁺², and Fe⁺²;
N is a trivalent metal cation selected between Al⁺³, Fe⁺³, and Cr⁺³; A is a mono-, di- or trivalent anion which decomposes at temperatures between 300-700 °C giving a volatile gas;
m and n have values in such way that the ratio m/n is between 1 and 6;
b has a value between 1 and 10;
when A is an monovalent anion, a = n;
when A is a divalent anion a = ½;
when A is a trivalent anion a = 1/3.

The layered double hydroxide precursor is calcined at temperatures between 300 and 700 °C and/or hydrated with an amount of water between 20-40 wt% previously to their use as catalyst.

As an example of these materials, the hydrotalcite, is aluminium magnesium hydroxide of laminar structure of formula Mg₆Al₂(OH)₁₆(CO₃)₂ 4H₂O which can be prepared with differed Al/Mg molar ratios. The calcinations of this material at temperatures above 450 °C leads to a Al-Mg mixed oxide (Mg₆Al₂(OH)₂).

The preparation of hydrotalcites are carried out starting from Mg(N03)2, Al(NO3)3, Na2CO3 and NaOH of determined concentrations according to methodologies previously reported (for instance, J. Catal. 148 (1994) 205). The catalysts can be prepared means the aid of ultrasound radiation during the precipitation step according the methodology previously reported (Corma et al., J. Catal.,225 (2004) 316). At this point and for achieving the active operating catalyst claimed here it is required to add water in the range between 20 to 40 wt % with respect to the amount of the mixed oxide and an aliphatic alcohol between 1 and 12 carbon atoms in a ratio between 0.5 to 8 g/g of catalyst.

### EXAMPLE 1

The catalyst (0.5g) (Al/Mg mixed oxide, with a molar ratio Mg/Al=3) was activated in situ previously to the reaction in a 10 mL two-necked flask reactor at 450 °C under N₂ flow during 6h. After this time, the catalyst was hydrated by adding a 36 wt% of CO₂ free water and different amounts of MeOH were added. Then 17.5 mmol (1.26g) of butanal and 6.8 mmol (1.03g) of campholenal were added into the reactor which was equipped with a stirrer, and a reflux condenser. The resultant slurry was heated at 74 °C during 2h under N₂ atmosphere. The conversion of campholenal and selectivity to Levosandal was dependent on the amount of MeOH added, as is showed in Table 1.

**Table 1**

| Methanol (g /g catalyst) | Conversion Campholenal % | Yield Levosandal % | Selectivity % |
|---|---|---|---|
| 0 | 55 | 41 | 74 |
| 1 | 75 | 76 | 98 |
| 3.4 | 66 | 63 | 95 |
| 8.0 | 59 | 44 | 75 |
| 12.0 | 50 | 36 | 73 |

### EXAMPLE 2

The catalyst (0.5g) (Al/Mg mixed oxide, with a molar ratio Mg/Al=3) was activated in situ previously to the reaction in a 10 mL two-necked flask reactor at 450 °C under N₂ flow during 6h. After this time, the catalyst was hydrated by adding a 36 %wt of CO₂ free water and different amounts of MeOH were added. Then 6.8 mmol (1.03g) of campholenal were added into the reactor which was equipped with a dropping funnel charged with 17.5 mmol (1.26g) of butanal, stirrer, and a reflux condenser. The resultant slurry was heated at 74 °C during 1.5 h under N₂ atmosphere, whereas the butanal was added at a rate of 0.8 mL/h. The conversion of campholenal and selectivity to Levosandal was dependent on the amount of MeOH added, as is showed in Table 2.

**Table 2**

| Methanol (g/gcatalyst) | Conversion Campholenal % | Yield Levosandal % | Selectivity % |
|---|---|---|---|
| 0 | 75 | 60 | 80 |
| 0.3 | 88 | 75 | 85 |
| 0.6 | 95 | 85 | 89 |
| 1.7 | 98 | 90 | 92 |
| 3.4 | 80 | 77 | 96 |

### EXAMPLE 3

The catalyst (0.5g) (Al/Mg mixed oxide, with a molar ratio Mg/Al=3) was activated in situ previously to the reaction in a 10 mL two-necked flask reactor at 450 °C under N₂ flow during 6h. After this time, the catalyst was hydrated by adding a 36 %wt of CO₂ free water and then 160 wt% of 2-propanol was added. Then 6.8 mmol (1.03g) of campholenal were added into the reactor which was equipped with a dropping funnel charged with 17.5 mmol (1.26g) of butanal, stirrer, and a reflux condenser. The resultant slurry was heated at 74 °C during 2h under N₂ atmosphere, whereas the butanal was added at a rate of 0.8 mL/h. The conversion of campholenal after 2h reaction time was 90 % with a selectivity to Levosandal of 95 %.

### EXAMPLE 4

The catalyst (1g) of a Al/Mg mixed oxide, with a molar ratio Mg/Al=2 prepared by the conventional method as described in (J. Catal. 148 (1994) 205) was activated in situ previously to the reaction in a 10 mL two-necked flask reactor at 450 °C under air flow during 6h and then under N₂ flow during 6h. After this the reactor was charged with, 76 mmol (4.56 g) of isopropanol and 2 mmol (0.412 g) of Levosandal. The reactor was equipped with stirrer and a reflux condenser and heated at 90 °C under N₂ atmosphere. After 24h reaction time a conversion of Levosandal of 90 % with 70 % selectivity to Bacdanol was obtained.

### EXAMPLE 5

The catalyst (0.75g) of a Al/Mg mixed oxide, with a molar ratio Mg/Al=2 prepared by sing ultrasound irradiation during the precipitation step as described by Corma et al., in J. Catal.,225 (2004) 316, was activated in situ previously to the reaction in a 10 mL two-necked flask reactor at 450 °C under air flow during 6h and then under N₂ flow during 6h. After this the reactor was charged with, 76 mmol (4.56 g) of isopropanol and 2 mmol (0.412 g) of Levosandal. The reactor was equipped with stirrer and a reflux condenser and heated at 90 °C under N₂ atmosphere. After 4h reaction time a conversion of Levosandal of 99 % with 70 % selectivity to Bacdanol was obtained.

## Claims

1. A process for producing 2-ethyl-4-(2,2,3-trimethyl-3-cyclopenten-1-yl)-2-butenal (Levosandal) useful as perfume or intermediate for perfume with high yield and selectivity by the cross-aldol condensation between campholenal and butanal in the presence of a controlled amount of a primary or secondary aliphatic alcohol with 1 to 12 carbon atoms using a heterogeneous bifunctional catalyst, wherein the controlled amount of an aliphatic alcohol is between 0.3 and 8g of aliphatic alcohol per gram of the catalyst.

2. A process according to claim 1 wherein the heterogeneous catalyst is selected from the group consisting of mixed oxides or hydrated mixed oxides derived of layered double hydroxides (LDH) of divalent and trivalent metals (M⁺²/N⁺³) of formula:
MₘNₙ(OH)₂ₘ₊₂ₙ(A)ₐbH₂O,
where M is a divalent metal cation selected from the group consisting of Mg⁺², Ni⁺², Zn⁺², Sn⁺², Fe⁺²;
N is a trivalent metal cation selected from the group consisting of Al⁺³, Fe⁺³, Cr⁺³;
A is a mono-, di- or trivalent anion which decomposes at temperatures between 300-700 °C giving a volatile gas;
m and n have values wherein the ratio m/n is between 1 and 6;
b has a value between 1 and 10;
when A is an monovalent anion, a =n,
when A is a divalent anion a= ½,
when A is a trivalent anion a= 1/3.

3. A process according to any one of claims 1 or 2 wherein the catalyst is a mixed oxide selected from a heterogeneous mixed oxides comprising divalent and trivalent metals with a molar ratio M⁺²/M⁺³ between 1 and 6.

4. A process according to any one of claims 1 to 3 wherein the catalyst is a mixed oxide selected from a heterogeneous mixed oxides comprising aluminium and magnesium derived from hydrotalcites with a molar ratio Mg/Al between 1 and 6.

5. A process according to any one of claims 1 to 4 wherein the catalyst is a mixed oxide which has been submitted to a calcination process at a temperature between 40-700°C.

6. A process according to any one of claims 1 to 5 wherein the catalyst has been submitted to a partial hydration process by adding between 20 to 40% of water with respect to the amount of the mixed oxide.

7. A process according to any one of claims 1 to 6 wherein the synthesis of 2-ethyl-4-(2,2,3-trimethyl-3-cyclopenten-1-yl)-2-butenal is performed by mixing the reactants with a heterogeneous catalyst which has been treated previously by adding a controlled amount of an aliphatic alcohol.

8. A process according to any one of claims 1 to 7 wherein the reaction between campholenal and butanal is performed using a molar ratio campholenal/butanal of 1:1 to 1:10.

9. A process according to any one of claims 1 to 8 wherein the amount of catalyst can be between 1 and 40% with respect the total amount of reactants.

10. A process according to any one of claims 1 to 9 wherein the condensation can be carried out by mixing the reactants to react with each other or by dropping the butanal over the mixture campholenal-catalyst slurry to gradually react with each of other.

11. A process according to any one of claims 1 to 10 wherein the cross-aldol condensation is carried out under inert atmosphere, at atmospheric pressure or in autoclave reactor at a pressure between 101325 and 2026500 Pascals (Pa) (between 1 and 20 atm) and a temperature between 25 and 150°C.

## Patentansprüche

1. Ein Prozess zur Herstellung von 2-Ethyl-4-(2,2,3-trimethyl-3-cyclopenten-1-yl)-2-butenal (Levosandal), das als Parfüm oder Zwischenprodukt für ein Parfüm nützlich ist, mit hohem Ertrag und Trennvermögen durch die Cross-Aldolkondensation zwischen Campholenal und Butanal in Gegenwart einer kontrollierten Menge eines primären oder sekundären aliphatischen Alkohols mit 1 bis 12 Kohlenstoffatomen, unter Verwendung eines heterogenen bifunktionalen Katalysators, wobei die kontrollierte Menge eines aliphatischen Alkohols zwischen 0,3 und 8g des aliphatischen Alkohols pro Gramm des Katalysators liegt.

2. Ein Prozess gemäß Anspruch 1, wobei der heterogene Katalysator ausgewählt ist aus der Gruppe bestehend aus Mischoxiden oder hydrierten Mischoxiden, abgeleitet von geschichteten Doppelhydroxiden (LDH) divalenter und trivalenter Metalle (M⁺²/N⁺³) der Formel:
MₘNₙ(OH)₂ₘ₊₂ₙ(A)ₐbH₂0,
wobei M ein divalentes Metallkation ist, ausgewählt aus der Gruppe bestehend aus Mg⁺², Ni⁺², Zn⁺², Sn⁺², Fe⁺²;
N ein trivalentes Metallkation ist, ausgewählt aus der Gruppe bestehend aus Al⁺³, Fe⁺³, Cr⁺³;
A ein mono-, di- oder trivalentes Anion ist, das bei Temperaturen zwischen 300-700°C zerfällt und zu einem flüchtigen Gas wird;
m und n Werte aufweisen, bei denen das m/n-Verhältnis zwischen 1 und 6 liegt;
b einen Wert zwischen 1 und 10 hat;
wenn A ein monovalentes Anion a =n ist
wenn A ein divalentes Anion a= ½ ist
wenn A ein trivalentes Anion a= 1/3 ist.

3. Ein Prozess gemäß eines der Ansprüche 1 oder 2, wobei der Katalysator ein Mischoxid ist, ausgewählt aus heterogenen Mischoxiden, bestehend aus divalenten und trivalenten Metallen mit einem Molverhältnis M⁺²/M⁺³ zwischen 1 und 6.

4. Ein Prozess gemäß eines der Ansprüche 1 bis 3, wobei der Katalysator ein Mischoxid ist, ausgewählt aus heterogenen Mischoxiden, bestehend aus Aluminium und Magnesium, abgeleitet aus Hydrotalciten mit einem Molverhältnis Mg/Al zwischen 1 und 6.

5. Ein Prozess gemäß eines der Ansprüche 1 bis 4, wobei der Katalysator ein Mischoxid ist, das einem Calcinierungsprozess bei einer Temperatur zwischen 40-700°C unterzogen wurde.

6. Ein Prozess gemäß eines der Ansprüche 1 bis 5, wobei der Katalysator einem teilweisen Hydrierungsprozess, durch Hinzufügen von zwischen 20 und 40% Wasser im Verhältnis zur Mischoxidmenge, unterzogen wird.

7. Ein Prozess gemäß eines der Ansprüche 1 bis 6, wobei die Synthese von 2-Ethyl-4-(2,2,3-trimethyl-3-cyclopenten-1-yl)-2-butenal durch die Mischung der Reaktionsmittel mit einem heterogenen Katalysator durchgeführt wird, der zuvor durch Hinzufügen einer kontrollierten Menge eines aliphatischen Alkohols behandelt wurde.

8. Ein Prozess gemäß eines der Ansprüche 1 bis 7, wobei die Reaktion zwischen Campholenal und Butanal unter Verwendung eines Molverhältnisses Campholenal/Butanal von 1:1 zu 1:10 ausgeführt wird.

9. Ein Prozess gemäß eines der Ansprüche 1 bis 8, wobei die Menge des Katalysators zwischen 1 und 40%, in Bezug auf die Gesamtmenge der Reaktionsmittel, liegt.

10. Ein Prozess gemäß eines der Ansprüche 1 bis 9, wobei die Kondensation durch das Mischen der Reaktionsmittel ausgeführt werden kann, um miteinander zu reagieren, oder durch Auftröpfeln des Butanal auf das Gemisch des Campholenal-Katalysator-Breis, damit diese sukzessive miteinander reagieren.

11. Ein Prozess gemäß eines der Ansprüche 1 bis 10, wobei die Cross-Aldolkondensation unter inerter Atmosphäre, bei Umgebungsdruck oder in einem Autoklavreaktor bei einem Druck zwischen 101325 und 2026500 Pascal (Pa) (zwischen 1 und 20 Atm) und einer Temperatur zwischen 25 und 150°C, ausgeführt wird.

## Revendications

1. Un procédé de production de 2-éthyle-4-(2,2,3-triméthyle-3-cyclopentène-1-yl)-2-buténal (Levosandal) utile en tant que parfum ou intermédiaire pour parfum avec un rendement et une sélectivité élevés par la condensation inter-aldol entre campholénal et butanal en présence d'une quantité contrôlée d'un alcool aliphatique primaire ou secondaire avec 1 à 12 atomes de carbone au moyen d'un catalyseur bifonctionnel hétérogène, où la quantité contrôlée d'un alcool aliphatique se situe entre 0,3 et 8g d'alcool aliphatique par gramme de catalyseur.

2. Un procédé selon la Revendication 1 où le catalyseur hétérogène est sélectionné dans le groupe se composant d'oxydes mixtes ou oxydes mixtes hydratés dérivés d'hydroxydes doubles en couches (LDH) de métaux divalents ou trivalents (M⁺²/N⁺³) de la formule :
MₘNₙ(OH)₂ₘ₊₂ₙ(A)ₐbH₂0,
où M est un cation métallique divalent sélectionné dans le groupe se composant de Mg⁺², Ni⁺², Zn⁺², Sn⁺², Fe⁺²,
N est un cation métallique trivalent sélectionné dans le groupe se composant de Al⁺³, Fe⁺³, Cr⁺³,
A est un anion mono-, di- ou trivalent qui se décompose à des températures entre 300 et 700°C de façon à donner un gaz volatile,
m et n possèdent des valeurs où le rapport m/n se situe entre 1 et 6,
b possède une valeur entre 1 et 10,
lorsque A est un anion monovalent, a = n,
lorsque A est un anion divalent a = ½,
lorsque A est un anion trivalent a = 1/3.

3. Un procédé selon l'une quelconque des Revendications 1 ou 2 où le catalyseur est un oxyde mixte sélectionné parmi des oxydes mixtes hétérogènes comprenant des métaux divalents ou trivalents avec un rapport molaire M⁺²/M⁺³ entre 1 et 6.

4. Un procédé selon l'une quelconque des Revendications 1 à 3 où le catalyseur est un oxyde mixte sélectionné parmi des oxydes mixtes hétérogènes comprenant aluminium et magnésium dérivés d'hydrotalcites avec un rapport molaire Mg/Al entre 1 et 6.

5. Un procédé selon l'une quelconque des Revendications 1 à 4 où le catalyseur est un oxyde mixte qui a été soumis à un procédé de calcination à une température entre 40 et 700°C.

6. Un procédé selon l'une quelconque des Revendications 1 à 5 où le catalyseur a été soumis à un procédé d'hydratation partielle par l'ajout de 20 à 40% d'eau par rapport à la quantité d'oxyde mixte.

7. Un procédé selon l'une quelconque des Revendications 1 à 6 où la synthèse de 2-éthyle-4-(2,2,3-triméthyle-3-cyclopentène-1-yl)-2-buténal est exécutée par le mélange des réactifs avec un catalyseur hétérogène qui a été traité antérieurement par l'ajout d'une quantité contrôlée d'un alcool aliphatique.

8. Un procédé selon l'une quelconque des Revendications 1 à 7 où la réaction entre campholénal et butanal est réalisée au moyen d'un rapport molaire campholénal/butanal de 1:1 à 1:10.

9. Un procédé selon l'une quelconque des Revendications 1 à 8 où la quantité de catalyseur peut se situer entre 1 et 40% par rapport à la quantité totale de réactifs.

10. Un procédé selon l'une quelconque des Revendications 1 à 9 où la condensation peut être réalisée par le mélange des réactifs de façon à réagir les uns avec les autres ou par le versement du butanal sur le mélange de boue campholénal-catalyseur de façon à réagir graduellement l'un avec l'autre.

11. Un procédé selon l'une quelconque des Revendications 1 à 10 où la condensation inter-aldol est réalisée sous atmosphère inerte, à une pression atmosphérique ou dans un réacteur autoclave à une pression entre 101325 et 2026500 Pascals (Pa) (entre 1 et 20 atm) et à une température entre 25 et 150°C.
